# EUROPEAN PATENT APPLICATION

(11) **EP 4 306 084 A1**
(43) Date of publication of application: **17.01.2024**
(21) Application number: 23182668.6
(22) Date of filing: 30.06.2023
(51) Int. Cl.: A61F 2/95

(54) **DUAL GUIDEWIRE DELIVERY SYSTEM**

(30) Priority: 12.07.2022 US 202263388444 P
(71) Applicant: Medtronic Vascular Inc., Santa Rosa, CA 95403 (US)
(72) Inventor: PATEL, Manthan P., Santa Rosa, CA (US); PERKINS, Keith D., Santa Rosa, CA (US); MURRAY III, Robert J., Santa Rosa, CA (US); CLAESSENS, Steven M., Santa Rosa, CA (US); KING, Haley M., Santa Rosa, CA (US); ZAVER, Nrupen N., Santa Rosa, CA (US)
(74) Representative: Zimmermann & Partner Patentanwälte mbB

(57) **Abstract**

In one aspect, the present disclosure provides a method including positioning a first guidewire within a primary guidewire lumen of a multi-lumen tapered tip. A second guidewire is positioned within a secondary guidewire lumen and a shared guidewire lumen of the multi-lumen tapered tip, the second guidewire preventing the first guidewire from entering into the shared guidewire lumen. A delivery system including the multi-lumen tapered tip is advanced over the second guidewire. By advancing the delivery system over a single guidewire, any possibility of entanglement of multiple guidewires during advancement of the delivery system is eliminated. The method further includes a guidewire exchange including releasing the second guidewire from the shared guidewire lumen, advancing the first guidewire through and out of the shared guidewire lumen, and advancing the delivery system over the first guidewire to a deployment location.

## Description

### FIELD

The present technology is generally related to medical device systems and methods.

### BACKGROUND

Aneurysms and/or dissections may occur in blood vessels, and most typically occur in the aorta and peripheral arteries. Depending on the region of the aorta involved, the aneurysm may extend into areas having vessel bifurcations or segments of the aorta from which smaller "branch" arteries extend.

The aneurysmal region of the aorta can be bypassed by use of an endoluminally delivered tubular exclusion device, e.g., by a stent graft placed inside the vessel spanning the aneurysmal portion of the vessel, to seal off the aneurysmal portion from further exposure to blood flowing through the aorta. The use of stent grafts to internally bypass, within the aorta or flow lumen, the aneurysmal site, is not without challenges.

In particular, care must be taken so that critical branch vessels are not covered or occluded by the stent graft yet the stent graft must seal against the aorta wall and provide a flow conduit for blood to flow past the aneurysmal site. Where the aneurysm is located immediately adjacent to the branch vessels, there is a need to deploy the stent graft in a location which partially or fully extends across the location of the origin of the branch vessels from the aorta to ensure sealing of the stent graft to the artery wall.

To accommodate the branch vessels, a main vessel stent graft having a fenestration or opening in a side wall thereof may be used. The main vessel stent graft is positioned to align its fenestration with the ostium of the branch vessel. To positionally align the main vessel stent graft, a primary guidewire is located within the aorta and a secondary guidewire is located within the side branch, the second guidewire being pre-wired within the fenestration of the main stent graft. The delivery system is advanced to the deployment location over the first and second guidewires and then deployed.

In some cases, the main vessel stent graft is supplemented by another stent graft, often referred to as a branch stent graft. The branch stent graft is deployed through the fenestration into the branch vessel to provide a conduit for blood flow into the branch vessel.

### SUMMARY

The techniques of this disclosure generally relate to an assembly having a multi-lumen tapered tip. The multi-lumen tapered tip includes a primary guidewire lumen, a secondary guidewire lumen, a shared guidewire lumen, and a first second guidewire junction at an intersection of the primary guidewire lumen, the secondary guidewire lumen, and the shared guidewire lumen. The shared guidewire lumen is configured to received either a first guidewire or a second guidewire, but not both simultaneously. This insures that the first guidewire cannot be distally extended out of multi-lumen tapered tip until the second guidewire is selectively released from the shared guidewire lumen.

In one aspect, the present disclosure provides a method including positioning a first guidewire within a primary guidewire lumen of a multi-lumen tapered tip. A second guidewire is positioned within a secondary guidewire lumen and a shared guidewire lumen of the multi-lumen tapered tip, the second guidewire preventing the first guidewire from entering into the shared guidewire lumen. A delivery system including the multi-lumen tapered tip is advanced over the second guidewire. By advancing the delivery system over a single guidewire, any possibility of entanglement of multiple guidewires during advancement of the delivery system is eliminated. The method further includes a guidewire exchange including releasing the second guidewire from the shared guidewire lumen, advancing the first guidewire through and out of the shared guidewire lumen, and advancing the delivery system over the first guidewire to a deployment location.

In another aspect, the present disclosure provides an assembly including a multi-lumen tapered tip having a primary guidewire lumen, a first guidewire within the primary guidewire lumen, a shared guidewire lumen, and a second guidewire within the shared guidewire lumen. The second guidewire prevents the first guidewire from entering into the shared guidewire lumen.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a top perspective view of a dual lumen tapered tip in accordance with one embodiment.
FIG. 2 is a side view of the dual lumen tapered tip of FIG. 1 in accordance with one embodiment.
FIG. 3 is a top view of the dual lumen tapered tip of FIG. 1 in accordance with one embodiment.
FIG. 4 is a proximal end view of the dual lumen tapered tip of FIG. 1 in accordance with one embodiment.
FIG. 5 is a side view of a delivery system including the dual lumen tapered tip of FIG. 1 in accordance with one embodiment.
FIG. 6 is a cross-sectional view of the delivery system of FIG. 5 along the line VI-VI in accordance with one embodiment.
FIG. 7 is a cross-sectional view of a vessel assembly during deployment of a main vessel stent graft of the delivery system of FIGS. 5-6 in accordance with one embodiment.
FIG. 8 is a cross-sectional view of the delivery system of FIGS. 5-6 at a later stage of deployment of the main vessel stent graft in accordance with one embodiment.
FIG. 9 is a cross-sectional view of the delivery system of FIG. 8 at a later stage of deployment of the main vessel stent graft in accordance with one embodiment.
FIG. 10 is a cross-sectional view of the vessel assembly of FIG. 7 at a later stage of deployment of the main vessel stent graft in accordance with one embodiment.
FIG. 11 is a cross-sectional view of the vessel assembly of FIG. 10 at a later stage of deployment of the main vessel stent graft in accordance with one embodiment.
FIG. 12 is a cross-sectional view of the vessel assembly of FIG. 11 at a later stage of deployment of a branch stent graft in accordance with one embodiment.

### DETAILED DESCRIPTION

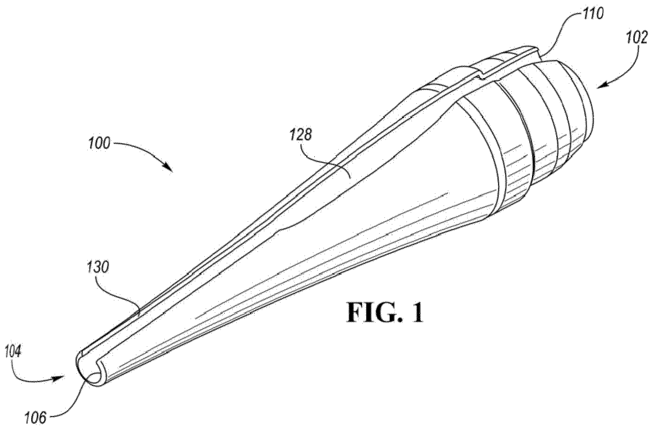

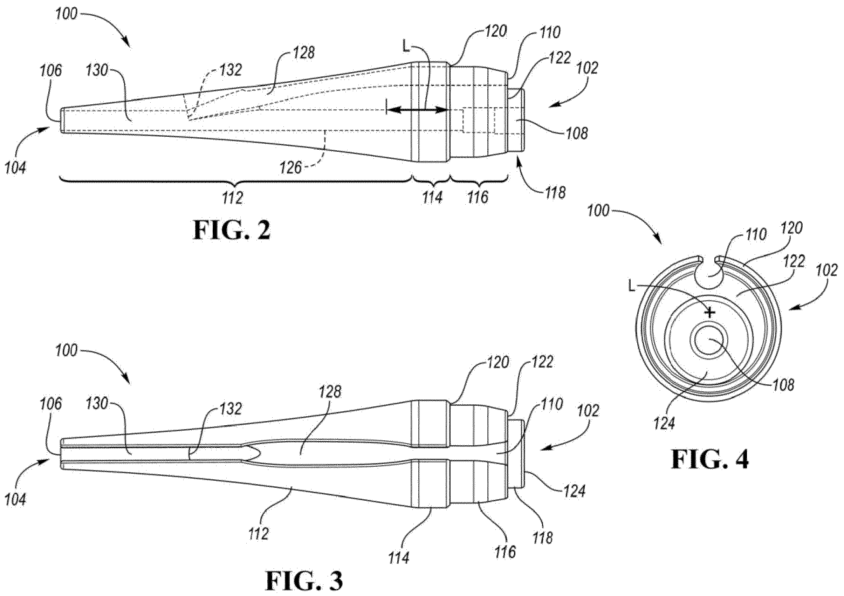

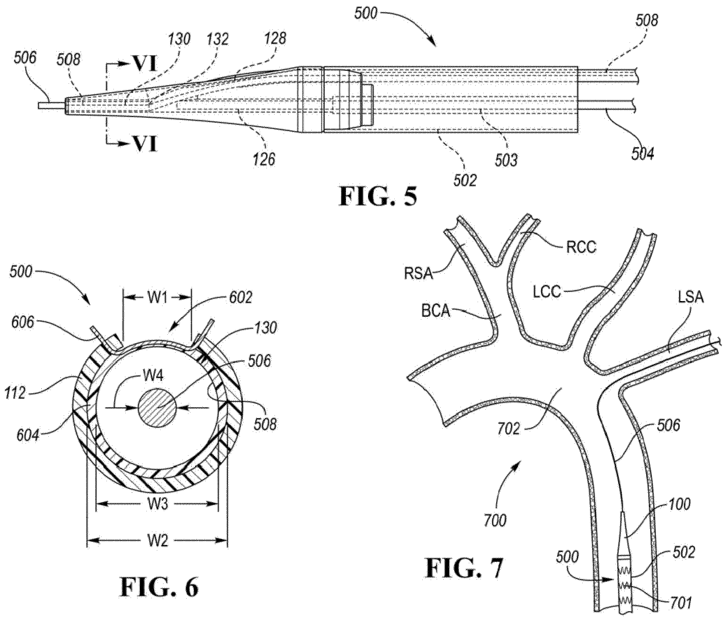

FIG. 1 is a top perspective view of a dual lumen tapered tip 100 in accordance with one embodiment. FIG. 2 is a side view of dual lumen tapered tip 100 of FIG. 1 in accordance with one embodiment. FIG. 3 is a top view of dual lumen tapered tip 100 of FIG. 1 in accordance with one embodiment. FIG. 4 is a proximal end view of dual lumen tapered tip 100 of FIG. 1 in accordance with one embodiment. FIG. 5 is a side view of a delivery system 500, sometimes called a dual guidewire delivery system, including dual lumen tapered tip 100 of FIG. 1 in accordance with one embodiment. FIGS. 2 and 5 illustrate internal features for ease of understanding however it is to be understood that the internal features may not be visible in actual use.

Referring now to FIGS. 1-5, dual lumen taper tip 100 is sometimes called a transcatheter delivery system nosecone for tracking with two guidewires without incurring wire-wrap. Dual lumen taper tip 100 has a proximal end 102 and a distal end 104. A distal guidewire lumen port 106, e.g., an opening, is located at distal end 104. A proximal primary guidewire lumen port 108 and a proximal secondary guidewire lumen port 110 e.g., openings, are located at proximal end 102.

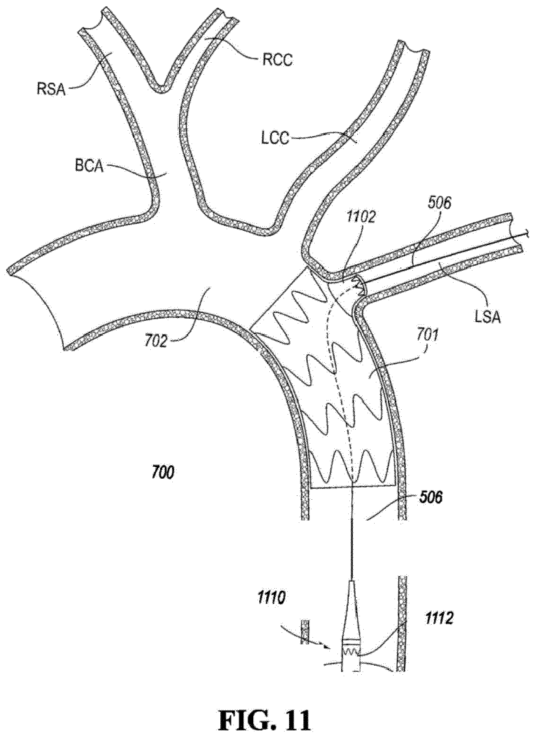

As used herein, the proximal end of a prosthesis such as a main vessel stent graft (discussed below in reference to FIG. 11) is the end closest to the heart via the path of blood flow whereas the distal end is the end furthest away from the heart during deployment. In contrast and of note, the distal end of delivery system 500 including dual lumen tapered tip 100 is usually identified to the end that is farthest from the operator/handle while the proximal end is the end nearest the operator/handle.

For purposes of clarity of discussion, as used herein, the distal end of delivery system 500 is the end that is farthest from the operator (the end furthest from the handle) while the distal end of the main vessel stent graft is the end nearest the operator (the end nearest the handle), i.e., the distal end of delivery system 500 and the proximal end of main vessel stent graft are the ends furthest from the handle while the proximal end of delivery system 500 and the distal end of main vessel stent graft are the ends nearest the handle. However, those of skill in the art will understand that depending upon the access location, the main vessel stent graft and delivery system 500 descriptions may be consistent or opposite in actual usage.

Between distal end 104 and proximal end 102, dual lumen tapered tip 100 includes a tapered portion 112, a middle portion 114, a sheath portion 116, and a proximal portion 118, respectively. As used herein, the longitudinal direction is the direction along the length or longitudinal axis of dual lumen tapered tip 100 and the transverse or radial direction is a direction perpendicular to the longitudinal direction.

Tapered portion 112 increases in diameter from and extends longitudinally between distal end 104 and cylindrical portion 114. Stated another way, tapered portion 112 tapers or flares from distal end 104 to middle portion 114. The taper of tapered portion 112 facilitate insertion and passage through a blood vessel of delivery system 500 including dual lumen tapered tip 100 as discussed further below.

Middle portion 114 has a uniform outer diameter in this embodiment and extends between tapered portion 112 and sheath portion 116. Middle portion 114 includes a transverse sheath stop surface 120 extending outward from sheath portion 116. A sheath 502 of delivery system 500 abuts sheath stop surface 120 in the loaded configuration as illustrated in FIG. 5.

Sheath portion 116 fits within and supports the distal end of sheath 502 as illustrated in FIG. 5. Sheath portion 116 includes a proximal taper to facilitate sliding of sheath 502 over sheath portion 116. However, in an embodiment, sheath portion 116 has a uniform outer diameter. Sheath portion 116 includes a transverse secondary guidewire port surface 122 extending outward from proximal portion 118. Proximal secondary guidewire lumen port 110 is located within secondary guidewire port surface 122.

Proximal portion 118 is located at proximal end 102 of dual lumen tapered tip 100 and has a uniform diameter in accordance with this embodiment. Proximal portion 118 includes a transverse primary guidewire port surface 124 at proximal end 102. Proximal primary guidewire lumen port 108 is located within primary guidewire port surface 124.

Dual lumen tapered tip 100 includes a primary guidewire lumen 126, a secondary guidewire lumen 128, and a shared guidewire lumen 130. The intersection of primary guidewire lumen 126, secondary guidewire lumen 128, and shared guidewire lumen 130 defines a first second guidewire junction 132, e.g., a Y-shaped junction.

Primary guidewire lumen 126 extends between proximal primary guidewire lumen port 108 and first second guidewire junction 132. Secondary guidewire lumen 128 extends between proximal secondary guidewire lumen port 110 and first second guidewire junction 132. In this embodiment, secondary guidewire lumen 128 curves inwards to meet primary guidewire lumen 126 and shared guidewire lumen 130 at first second guidewire junction 132. In other words, secondary guidewire lumen 128 redirect a second guidewire 506 from the outside towards the center of dual lumen tapered tip 100.

Shared guidewire lumen 130 extends between distal guidewire lumen port 106 and first second guidewire junction 132. In this embodiment, primary guidewire lumen 126 and shared guidewire lumen 130 lie upon a common straight line, are sometimes called linear, and extend in the longitudinal direction. In one embodiment, dual lumen tapered tip 100 is mounted on a tube 503 inserted into primary guidewire lumen 126.

In this embodiment, primary guidewire lumen 126 is dedicated to receiving a first guidewire 504 as illustrated in FIG. 5. Secondary guidewire lumen 128 is dedicated to receiving second guidewire 506 as illustrated in FIG. 5. As discussed further below, shared guidewire lumen 130 is configured to received either first guidewire 504 or second guidewire 506, but not both simultaneously. More particularly, when second guidewire 506 (and optionally a surrounding guide tube 508) is located within shared guidewire lumen 130 as illustrated in FIG. 5, second guidewire 506 (or the surrounding guide tube 508) acts as a stop to prevent first guidewire 504 from advancing and entering into shared guidewire lumen 130. This insures that first guidewire 504 cannot be distally extended out of dual lumen tapered tip 100 until second guidewire 506 is removed from shared guidewire lumen 130.

Paying particular attention to FIGS. 2, 4, to provide space for secondary guidewire lumen 128, primary guidewire lumen 126 may be offset from the central longitudinal axis L of dual lumen tapered tip 100. More particularly, primary guidewire lumen 126 is displaced below longitudinal axis L relative to secondary guidewire lumen 128. In contrast to having primary guidewire lumen 126 located at longitudinal axis L, by offsetting primary guidewire lumen 126, additional area may be provided for secondary guidewire lumen 128. This allows the overall dimensions of dual lumen tapered tip 100, sometimes called the profile, to be minimized thus increasing the range of anatomical applications and reducing trauma to the blood vessel during delivery.

Paying particular attention to FIGS. 1, 3, to further reduce the overall dimensions of dual lumen tapered tip 100, secondary guidewire lumen 128 may be an open trench, in contrast to a sealed lumen. By providing second guidewire lumen 128 as an open trench in contrast to a sealed lumen, the thickness of material that would otherwise be necessary to enclose second guidewire lumen 128 is avoided. This allows the overall dimensions of dual lumen tapered tip 100 to be further reduced thus further increasing the range of anatomical applications and reducing trauma to the blood vessel during delivery.

In accordance with this embodiment, the depth of secondary guidewire lumen 128 varies. Accordingly, as illustrated in FIGS. 1 and 3, the width of the opening of secondary guidewire lumen 128 varies as a function of the depth. However, in an embodiment, the depth and the width of secondary guidewire lumen 128 is constant and uniform.

In one embodiment, the diameter of second guidewire 506 is 0.035", see for example width W4 in FIG. 6. However, in other embodiments, the diameter of second guidewire 506 is reduced to less than 0.035", for example, is 0.018" or 0.014". By reducing the diameter of second guidewire 506, the depth of secondary guidewire lumen 128 can be reduced thus reducing the profile of dual lumen tapered tip 100.

Although both an offset primary guidewire lumen 126 and open trench secondary guidewire lumen 128 are discussed above, in an embodiment, dual lumen tapered tip 100 includes an offset primary guidewire lumen 126 or an open trench secondary guidewire lumen 128, but not both. In yet another embodiment, dual lumen tapered tip 100 includes an axially aligned primary guidewire lumen 126 and an initially sealed second guidewire lumen 128.

FIG. 6 is a cross-sectional view of delivery system 500 of FIG. 5 along the line VI-VI in accordance with one embodiment. Referring now to FIGS. 5 and 6 together, shared guidewire lumen 130 is an open trench in accordance with this embodiment. A width W1 of an opening 602 of shared guidewire lumen 130 is less than a width W2 of a body 604 of shared guidewire lumen 130. Shared guidewire lumen 130 is sometimes called an undercut trench in that the cross sectional width of shared guidewire lumen 130 increases from width W1 of opening 602 to width W2 of body 604. Stated another way, shared guidewire lumen 130 is a circular trench in accordance with this embodiment.

In the initial deployment configuration as illustrated in FIGS. 5 and 6, second guidewire 506 and a guide tube 508 are located within secondary guidewire lumen 128 and shared guidewire lumen 130. Second guidewire 506 is located within guide tube 508, sometimes called a PEEK lumen. Guide tube 508 has a width W3, sometimes called an outer diameter, approximately equal to width W2 of body 604 of shared guidewire lumen 130 and greater than width W1 of opening 602. Accordingly, guide tube 508 cannot pass through opening 602 and is trapped within shared guidewire lumen 130.

Accordingly, when guide tube 508 is located within shared guidewire lumen 130, second guidewire 506 is also trapped within shared guidewire lumen 130. However, second guidewire 506 has a width W4, sometimes called an outer diameter, less than width W1 of opening 602. Accordingly, upon retraction of guide tube 508 from shared guidewire lumen 130, second guidewire 506 is released and can pass through opening 602 and out of shared guidewire lumen 130.

Although use of guide tube 508 to control selective release of second guidewire 506 is illustrated and discussed, in other embodiments, other mechanisms to control selective release of second guidewire 506 from shared guidewire lumen 130 are used. For example, a wire 606, sometimes called a trigger wire, can be laced over opening 602 of shared guidewire lumen 130. Wire 606 can be retracted thus freeing opening 602 of shared guidewire lumen 130 and releasing secondary guidewire 506. Although both wire 606 and guide tube 508 are illustrated in FIG. 6 and can be used together in an embodiment, typically wire 606 or guide tube 508 are present but not both.

FIG. 7 is a cross-sectional view of a vessel assembly 700 during deployment of a main vessel stent graft 701 of delivery system 500 of FIGS. 5-6 in accordance with one embodiment. Referring to FIGS. 5-7 together, the thoracic aorta 702 has numerous arterial branches. The arch of the aorta 702 has three major branches extending therefrom, all of which usually arise from the convex upper surface of the arch. The brachiocephalic artery BCA originates anterior to the trachea. The brachiocephalic artery BCA divides into two branches, the right subclavian artery RSA (which supplies blood to the right arm) and the right common carotid artery RCC (which supplies blood to the right side of the head and neck). The left common carotid artery LCC artery arises from the arch of the aorta 702 just to the left of the origin of the brachiocephalic artery BCA. The left common carotid artery LCC supplies blood to the left side of the head and neck. The third branch arising from the aortic arch, the left subclavian artery LSA, originates behind and just to the left of the origin of the left common carotid artery LCC and supplies blood to the left arm.

However, a significant proportion of the population has only two great branch vessels coming off the aortic arch while others have four great branch vessels coming of the aortic arch. Accordingly, although a particular anatomical geometry of the aortic arch is illustrated and discussed, in light of this disclosure, those of skill in the art will understand that the geometry of the aortic arch has anatomical variations and that the various structures as disclosed herein would be modified accordingly.

Aneurysms, dissections, penetrating ulcers, intramural hematomas and/or transections, generally referred to as a diseased region of the aorta 702, may occur in the aortic arch and the peripheral arteries BCA, LCC, LSA. For example, thoracic aortic aneurysms include aneurysms present in the ascending thoracic aorta, the aortic arch, and one or more of the branch arteries BCA, LCC, LSA that emanate therefrom. Thoracic aortic aneurysms also include aneurysms present in the descending thoracic aorta and branch arteries that emanate therefrom. Accordingly, the aorta 702 as illustrated in FIG. 7 has a diseased region similar to any one of those discussed above which will be bypassed and excluded as discussed below.

Initially , second guide wire 506 is introduced via femoral access. In one particular embodiment, second guidewire 506 is inserted into the femoral artery and routed up through the abdominal aorta, into the thoracic aorta. Second guidewire 506 is snared or otherwise moved into the left subclavian artery LSA, e.g., the physician has cannulated the left subclavian artery LSA.

Delivery system 500 is introduced via femoral access and is advanced, sometimes called tracked, into aorta 702 over second guidewire 506. Delivery system 500 is positioned at the desired location near the left subclavian artery LSA.

During advancement of delivery system 500 over second guidewire 506, first guidewire 504 is blocked and prevented from being advanced out of delivery system 500 by second guidewire 506 as discussed above. Alternatively, first guidewire 504 may not be inserted in the delivery system during this stage of tracking, but may be inserted later when second guidewire 506 exits the tapered tip (or just prior). In one embodiment, second guidewire 506 is less stiff than first guidewire 504, and thus placement of second guidewire 506 as well as advancement of delivery system 500 is simplified.

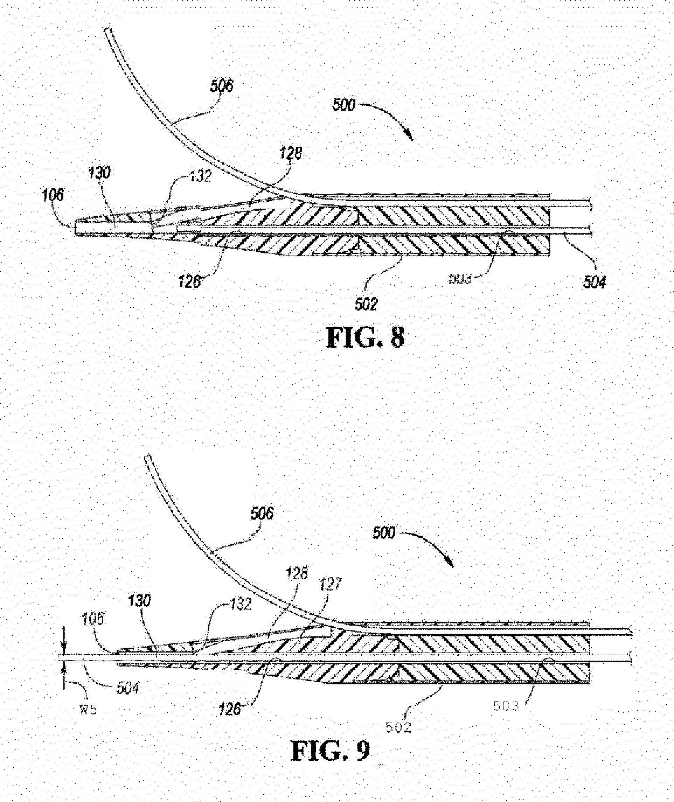

FIG. 8 is a cross-sectional view of delivery system 500 of FIGS. 5-6 at a later stage of deployment of main vessel stent graft 701 in accordance with one embodiment. Referring now to FIGS. 5-8 together, upon reaching the landing zone near the left subclavian artery LSA, second guidewire 506 is exchanged for first guidewire 504. More particularly, guide tube 508 is withdrawn thus releasing second guidewire 506 from shared guidewire lumen 130 and secondary guidewire lumen 128 as illustrated in FIG. 8 and allowing the physician to orient second guidewire 506 towards the left subclavian artery LSA, through which the second guidewire 506 already extends. This frees shared guidewire lumen 130 to allow first guidewire 504 to be advanced therein as illustrated in FIG. 9. Stated another way, second guidewire 506 is unsheathed from guide tube 508 and released from shared guidewire lumen 130 allowing first guidewire 504 to push forward.

As set forth above, delivery system 500 is first advanced over second guidewire 506 to be near the left subclavian artery LSA. By advancing delivery system 500 over a single guidewire, i.e., second guidewire 506, any possibility of entanglement of multiple guidewires during advancement of delivery system 500 is eliminated as compared to advancing delivery system 500 over two or more guidewires. Stated another way, by avoiding entanglement of multiple guidewires, untangling the wires including manipulating delivery system 500 by torqueing and axially moving delivery system 500 back and forth is avoided. This shortens the procedure time, reduces radiation exposure, and reduces embolic risk to the patient. Reducing wire wrap in the arch may be particularly important, since any manipulations used to resolve wire wrap may raise the risk of stroke. Further, second guidewire 506 is more flexible than first guidewire 504 thus simplifying the procedure.

FIG. 9 is a cross-sectional view of delivery system 500 of FIG. 8 at a later stage of deployment of main vessel stent graft 701 in accordance with one embodiment. Referring now to FIG. 9, first guidewire 504 is advance through and extends distally from shared guidewire lumen 130. First guidewire 504 is advanced into the aortic arch. First guidewire 504 is preventing from being released from shared guidewire lumen 130 by a molding features 127 between primary guidewire lumen 126 and secondary guidewire lumen 128 as illustrated in FIG. 9.

In another embodiment, first guidewire 504 has a width W5 greater than width W1 of opening 602 and less than or approximately equal to width W2 of body 604 of shared guidewire lumen 130. Width W5 is illustrated in FIG. 9 and width W1 of opening 602 and width W2 of body 604 are illustrated in FIG. 6. Accordingly, when first guidewire 504 is located within shared guidewire lumen 130, first guidewire 504 is trapped within shared guidewire lumen 130. For example, when second guidewire 506 has width W4 smaller than width W5 of first guidewire 504, second guidewire 506 can escape shared guidewire lumen 130 while first guidewire 504 is prevented from escaping from shared guidewire lumen 130 even absent molding feature 127. In this embodiment, an interference fit between first guidewire 504 and shared guidewire lumen 130 insures that first guidewire 504 exits distal guidewire lumen port 106 and does not get pulled up and released through opening 602. For example, first guidewire 504 has width W5 of 0.035" and second guidewire 506 has width W4 less than width W5, e.g., less than 0.035" such as 0.018" or 0.014".

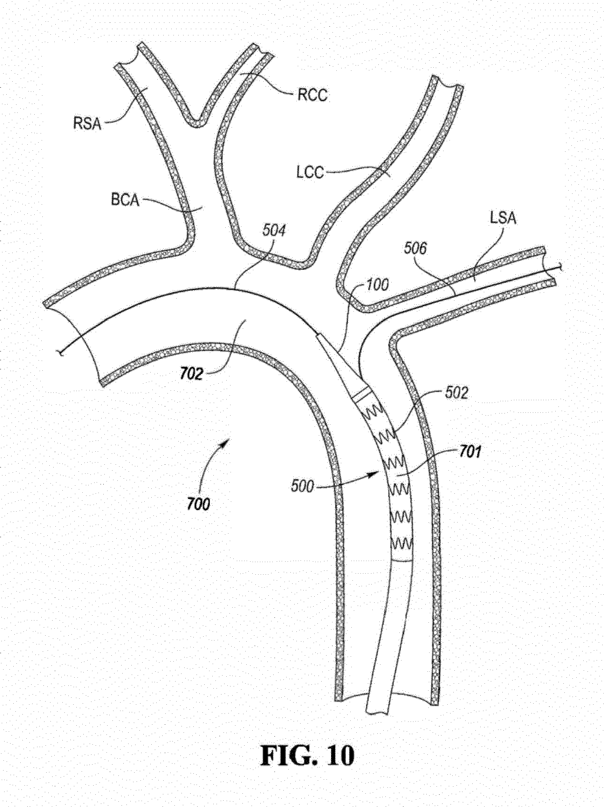

FIG. 10 is a cross-sectional view of vessel assembly 700 of FIG. 7 at a later stage of deployment of main vessel stent graft 701 in accordance with one embodiment. Once first guidewire 504 is placed into the aortic arch as illustrated in FIG. 10 and discussed above regarding FIG. 9, delivery system 500 is advanced over guidewires 504, 506 and rotationally aligned to be in the deployment location. Delivery system 500 is advanced a minimal distance thus minimizing and essentially eliminating any possibility of entanglement of guidewires 504, 506.

FIG. 11 is a cross-sectional view of vessel assembly 700 of FIG. 10 at a later stage of deployment of main vessel stent graft 701 in accordance with one embodiment. Referring now to FIGS. 10 and 11 together, once delivery system 500 is located at the deployment location, sheath 502 is retracted and main vessel stent graft 701 having a mobile external coupling 1102 (MEC 1102) is deployed. While a mobile external coupling is shown, the stent graft 701 may alternatively have a standard coupling or a fenestration (e.g., opening). Second guidewire 506 extends through mobile external coupling 1102 and into the left subclavian artery LSA ensuring rotational alignment of mobile external coupling 1102 with the left subclavian artery LSA. A main vessel stent graft having a mobile external coupling including deployment thereof similar to main vessel stent graft 701 and mobile external coupling 1102 is described in Bruszewski et al., U.S. Patent No. 9,839,542, issued on December 12, 2017, which is herein incorporated by reference in its entirety.

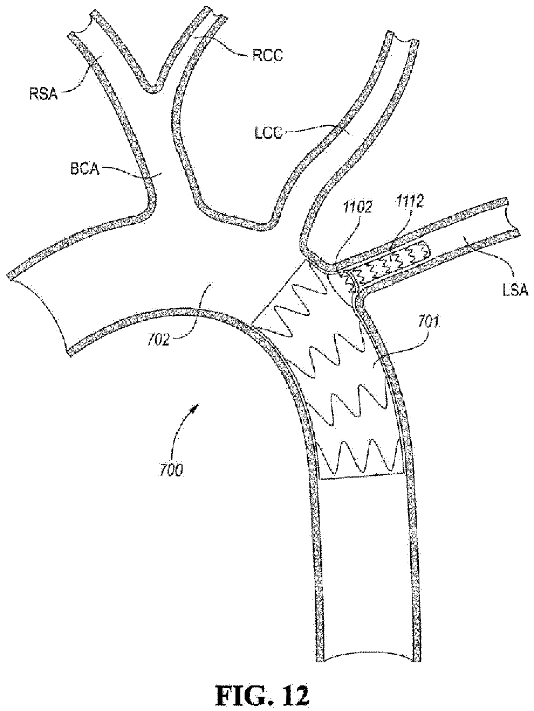

FIG. 12 is a cross-sectional view of vessel assembly 700 of FIG. 11 at a later stage of deployment of a branch stent graft 1112 in accordance with one embodiment. Referring now to FIGS. 11 and 12 together, a delivery system 1110 including branch stent graft 1112 is advanced over second guidewire 506, through mobile external coupling 1102, and into the left subclavian artery LSA. Branch stent graft 1112 is deployed into and branches mobile external coupling 1102 into the left subclavian artery LSA as illustrated in FIG. 12. While delivery system 1110 is shown as introduced via femoral access, it may alternatively be introduced via supra-aortic access (e.g., with corresponding adjustments to the loading of the stent graft 1112).

Although aorta 702 as the main vessel and the left subclavian artery LSA as the branch vessel are discussed above, in other embodiment, a main vessel stent graft is deployed in other main vessels and associated branch vessels in a similar manner using dual lumen tapered tip 100. For example, for iliac branch therapy delivery, second guidewire 506 is used to facilitate cannulation of the internal iliac artery.

Further, although dual lumen tapered tip 100 is discussed as being used with two guidewires 504, 506, in other embodiments, a similar tapered tip is used to selectively advance three or more guidewires. Generally, tapered tip 100 is sometimes called a multi-lumen tapered tip and has two or more lumens therein. In such embodiments, there may be multiple guidewires in a retracted position during initial tracking, similar to first guidewire 504, such that tracking can still occur over a single wire to avoid wire wrap. There may also be multiple secondary lumens (e.g., trenched lumens) configure to allow the wires to be released at a later stage of the deployment to orient towards a branch vessel ostia. For example, a third wire may be retracted during an initial tracking, similar to FIG. 7, and then extended outward once at the desired location towards the brachiocephalic artery BCA or the left common carotid artery LCC. The third wire could then be advanced into the branch artery via manual manipulation or could be snared.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

For example, further embodiments of the invention may be as follows:

According to a first further embodiment, there is provided an assembly which comprises a multi-lumen tapered tip, wherein said multi-lumen tapered tip comprises: a primary guidewire lumen; a first guidewire within the primary guidewire lumen; a shared guidewire lumen; and a second guidewire within the shared guidewire lumen, the second guidewire preventing the first guidewire from entering into the shared guidewire lumen.

According to a second further embodiment, there is provided the assembly of the first further embodiment, further comprising a guide tube within the shared guidewire lumen, the second guidewire being within the guide tube.

According to a third further embodiment, there is provided the assembly of the second further embodiment, wherein the shared guidewire lumen comprises a trench comprising an opening having a width.

According to a fourth further embodiment, there is provided the assembly of the third further embodiment, wherein the width of the opening is less than a width of the guide tube and greater than a width of the second guidewire.

According to a fifth further embodiment, there is provided the assembly of one of the first to fourth further embodiments, further comprising a secondary guidewire lumen, the second guidewire being within the secondary guidewire lumen.

According to a sixth further embodiment, there is provided the assembly of the fifth further embodiment, further comprising a first second guidewire junction at an intersection of the primary guidewire lumen, the secondary guidewire lumen, and the shared guidewire lumen.

According to a seventh further embodiment, there is provided a method comprising: positioning a first guidewire within a primary guidewire lumen of a multi-lumen tapered tip; and positioning a second guidewire within a secondary guidewire lumen and a shared guidewire lumen of the multi-lumen tapered tip, the second guidewire preventing the first guidewire from entering into the shared guidewire lumen.

According to an eighth further embodiment, there is provided the method of the seventh further embodiment, further comprising: advancing a delivery system comprising the multi-lumen tapered tip over the second guidewire; and releasing the second guidewire from the shared guidewire lumen.

According to a ninth further embodiment, there is provided the method of seventh or eighth further embodiment, further comprising: advancing the first guidewire through and out of the shared guidewire lumen; and advancing the delivery system over the first guidewire to a deployment location.

According to a tenth further embodiment, there is provided the method of one of the seventh to ninth further embodiments, further comprising deploying a main vessel stent graft from the delivery system, wherein the second guidewire extends through a coupling or fenestration of the main vessel stent graft.

According to an eleventh further embodiment, there is provided the method of the tenth further embodiment, further comprising: advancing a delivery system comprising a branch stent graft over the second guidewire and into the coupling or fenestration; and deploying the branch stent graft within the coupling or fenestration.

## Claims

1. An assembly comprising:
a multi-lumen tapered tip comprising:
a primary guidewire lumen;
a secondary guidewire lumen;
a shared guidewire lumen; and
a first second guidewire junction at an intersection of the primary guidewire lumen, the secondary guidewire lumen, and the shared guidewire lumen.

2. The assembly of Claim 1, wherein the multi-lumen tapered tip further comprises:
a distal guidewire lumen port, the shared guidewire lumen extending between the distal guidewire lumen port and the first second guidewire junction.

3. The assembly of Claim 1 or 2, wherein the multi-lumen tapered tip further comprises:
a proximal primary guidewire lumen port, the primary guidewire lumen extending between the proximal primary guidewire lumen port and the first second guidewire junction.

4. The assembly of one of Claims 1-3, wherein the multi-lumen tapered tip further comprises:
a proximal secondary guidewire lumen port, the secondary guidewire lumen extending between the proximal secondary guidewire lumen port and the first second guidewire junction.

5. The assembly of Claim 4 wherein the secondary guidewire lumen curves inward to the first second guidewire junction.

6. The assembly of one of Claims 1-5 wherein the primary guidewire lumen and the shared guidewire lumen are linear.

7. The assembly of one of Claims 1-6 wherein the primary guidewire lumen is offset from a central longitudinal axis of the multi-lumen tapered tip.

8. The assembly of one of Claims 1-7 wherein the shared guidewire lumen comprises an open trench.

9. The assembly of one of Claims 1-8 wherein the secondary guidewire lumen comprises an open trench.
